# EUROPÄISCHE PATENTSCHRIFT

(11) **EP 2 367 546 B1**
(45) Veröffentlichungstag und Bekanntmachung des Hinweises auf die Patenterteilung: **08.05.2013**
(21) Anmeldenummer: 09778412.8
(22) Anmeldetag: 08.09.2009
(51) Int. Cl.: A61K 31/167, A61K 31/192, A61P 23/02, A61P 29/00

(54) **KOMBINATION AUS LOKALANÄSTHETIKUM UND ANALGETIKUM ZUR LINDERUNG VON BRUSTSCHMERZ**
COMBINATION OF LOCAL ANESTHETIC AND ANALGESIC FOR RELIEVING BREAST PAIN
COMBINAISON D'UN ANESTHÉSIQUE LOCAL ET D'UN ANALGÉSIQUE POUR SOULAGER LES DOULEURS DE POITRINE

(30) Priorität: 22.12.2008 DE 102008064214
(43) Veröffentlichungstag der Anmeldung: 28.09.2011
(73) Patentinhaber: Klosterfrau Berlin Gmbh, 12277 Berlin (DE)
(72) Erfinder: GALÁN-SOÚSA, José, 13469 Berlin (DE)
(74) Vertreter: Von Rohr
(86) Internationale Anmeldenummer: PCT/EP2009/006523
(87) Internationale Veröffentlichungsnummer: WO 2010/072277

(56) Entgegenhaltungen:
- WO-A-02/24185
- LAMBERTZ COLLEEN K ET AL: "Premedication to reduce discomfort during screening mammography" RADIOLOGY, Bd. 248, Nr. 3, September 2008 (2008-09), Seiten 765-772, XP002556102 ISSN: 0033-8419
- KHAN H N ET AL: "Local anaesthetic and steroid combined injection therapy in the management of non-cyclical mastalgia" BREAST, Bd. 13, Nr. 2, April 2004 (2004-04), Seiten 129-132, XP002556103 ISSN: 0960-9776
- DUNN ET AL: "Chronic Regional Pain Syndrome, Type 1: Part I" AORN JOURNAL, ASSOCIATION OF OPERATING ROOM NURSES, DENVER, CO, US, Bd. 72, Nr. 3, 1. September 2000 (2000-09-01), Seiten 421-424,426,4, XP005639910 ISSN: 0001-2092

## Beschreibung

Die vorliegende Erfindung betrifft den Bereich der mit medizinischen, insbesondere mammographischen Untersuchungen einhergehenden Nebenwirkungen bzw. Beschwerden, wie Schmerzen, unangenehmen Druck- oder Kompressionsempfinduzigen, Befindlichkeisstörungen oder dergleichen der insbesondere weiblichen Brust.

Insbesondere betrifft die vorliegende Erfindung die Verwendung eines Lokal-anästhetikurns in Kombination mit einem Analgetikum zur Herstellung eines Arzneimittels zur prophylaktischen und/oder therapeutischen Behandlung von Brustschmerz (Schmerzen der Brust) insbesondere der weiblichen Brust, welcher mit medizinischen, insbesondere mammographischen Untersuchungen im Zusammenhang steht,

Des weiteren betrifft die vorliegende Erfindung eine topisch anwendbare Applikationsform, welche sich zur prophylaktischen oder therapeutischen Behandlung von Brustschmerz (Schmerzen der Brust), welcher durch medizinische, insbesondere mammographische Untersuchungen verursacht wird, eignet. Die Zusammensetzung nach der Erfindung enthält mindestens ein Lokalanästhetikum in Kombination mit mindestens einem Analgetikum.

Schließlich betrifft die vorliegende Erfindung ein Kit, welches mindestens eine topisch anwendbare Applikationsform mit mindestens einem Lokalanästhetikum und mindestens eine separate topisch anwendbare Applikationsform mit einem Analgetikum enthält.

Die Mammographie ist als Verfahren der Radiologie eine Röntgenuntersuchung üblicherweise der weiblichen, gegebenenfalls aber auch der männlichen Brust. Die Mammographie ist eine Methode zur Früherkennung von Brustkrebs, insbesondere des Mammakarzinoms, der häufigsten Krebserkrankung der Frau. Die Mammographie kann einerseits zur Früherkennung von Brustkrebs - beispielsweise im Rahmen von Screening-Untersuchungen - eingesetzt werden, andererseits aber auch zur weiteren Abklärung tumorverdächtiger Befunde, wie tastbaren Knoten oder dergleichen.

Die mammographische Untersuchung erfolgt dabei unter Verwendung spezieller Röntgengeräte, wobei jede Brust in der Regel aus zwei Richtungen (meist von oben und schräg seitlich) aufgenommen wird, Während der Untersuchung ist es erforderlich, daß die Brust zwischen einem Halter, welcher insbesondere auch als Filmhalter bzw. Kassettenhalter bezeichnet wird, und einer Plexiglasplatte der Apparatur eingespannt bzw. komprimiert wird, Hierdurch soll die Strahlendosis so gering wie möglich gehalten und das Gewebe zudem aufgefächert werden, so daß auf diese Weise ein größeres Gewebegebiet bei gleichzeitig verringerter Strahlendosis untersucht werden kann.

Ein großer Nachteil mammographischer Untersuchungen ist darin zu sehen, daß diese unangenehm bzw. mit Befindlichkeitsstörungen und Schmerzen verbunden sein können, was maßgeblich damit zusammenhängt, daß die weibliche Brust während der Untersuchung eingespannt bzw. komprimiert und damit gewissermaßen gequetscht werden muß. Die mit der Mammographie einhergehenden Schmerzen und Unannehmlichkeiten sind oftmals sogar ein Grund dafür, daß Frauen trotz medizinischem Erfordernis notwendige Mammographieuntersuchungen nicht durchführen lassen.

Im Stand der Technik sind bislang keine wirksamen Ansätze bekannt, welche zu einer signifikanten Linderung von mit mammographischen Untersuchungen einhergehenden Unannehmlichkeiten, Befindlichkeitsstörungen bzw. Schmerzen führen. Zwar kann die systemische Gabe von Analgetika in Betracht gezogen werden, jedoch ist die schmerzstillende Wirkung in bezug auf die weibliche Brust oftmals bei weitem nicht zufriedenstellend, und die systemische Gabe von starken Analgetika ist mitunter mit unerwünschten Nebenwirkungen verbunden.

Lambertz et al. : "Premedication to Reduce Discomfort during Screening Mammography", Radiology, 2008, 248, Seiten 765 bis 772*,* betrifft eine medizinische Anwendung, welche der Linderung von Beschwerden sowie der Verbesserung der Befindlichkeit bei mammographischen Untersuchungen dient. Bei der Medikation handelt es sich um ein Zweikomponentensystem, nämlich um eine topisch anwendbare lidocainhaltige Formulierung einerseits sowie ein oral einzunehmendes Analgetikum andererseits.

Die WO 02/24185 A2 betrifft ein Verfahren zur lokalen Schmerzstillung bei Sportunfällen, körperlichen Übergriffen, Arthritis, Rheumatismus, Kopfschmerzen oder Operationsschmerzen. Dabei handelt es sich um eine topisch applizierbare Zusammensetzung, welche Analgetika auf Basis von aliphatischen Polyaminen (biogenen Aminen) enthält. Bevorzugterweise werden dabei Putrescin, Spermin, Spermidin und Cadaverin eingesetzt. Um ein Eindringen der Wirkstoffe in die Haut zu ermöglichen, werden der Zusammensetzung chaotrope Substanzen, wie z. B. Harnstoff, zugesetzt. Optional kann die pharmazeutische Zusammensetzung auch Lidocain als Lokalanästhetikum enthalten

Khan et al.: "Local anaesthetic and steriod combined injection therapy in the management of non-cyclical mastalgia", The Breast, 2004, 13, Seiten 129 bis 132*,* betrifft eine kombinierte Injektionstherapie, welche ein Lokalanästhetikum und ein Steroid zur Behandlung nichtzyklischer Mastodynie umfasst. Betroffene Patientinnen erhalten dazu eine Injektion, welche als pharmazeutische Wirkstoffe Lidocain und Depomedron enthält und welche oberflächlich in den Bereich der Rippen in Brustnähe gespritzt wird.

Dunn :"Chronic Regional Pain Syndrome, Type 1: Part I", AORN JOURNAL, 2000, 72, Seiten 421 bis 449*,* stellt einen Übersichtsartikel über das komplexe regionale SchmerzSyndrom (CRPS) dar, wobei unter anderem Ursachen, Pathophysiologie, Anzeichen und Symptome, der Krankheitsverlauf sowie Behandlungsmethoden aufgeführt werden. Unter anderem wird in dem Fachartikel die Behandlung einer Frau dokumentiert, welcher im Rahmen einer CRPS-Erkrankung aufgrund von Brustschmerzen eine Injektion auf Basis eines Blockiermittels für das *Ganglion stellatum* in Kombination mit dem Lokalanästhetikum Bupivacain zur Schmerzlinderung verabreicht wurde.

Vor diesem Hintergrund besteht - insbesondere auch aufgrund der Häufigkeit von mammographischen Untersuchungen und den damit einhergehenden Befindlichkeitsstörungen bzw. Schmerzen - ein großer Bedarf, eine geeignete prophylaktische bzw. therapeutische Behandlung von mit diesen medizinischen Untersuchungen einhergehenden Brustschmerzen (Schmerzen der Brust) bereitzustellen, welche sich in effizienter Weise zur prophylaktischen bzw. therapeutischen Behandlung von Brustschmerz eignen und dabei nur geringe Nebenwirkungen aufweisen.

Somit liegt der vorliegenden Erfindung die Aufgabe zugrunde, eine neuartige Verwendung bzw. einen neuartigen prophylaktischen bzw. therapeutischen Ansatz bereitzustellen, welche bzw. welcher sich zur prophylaktischen und/oder therapeutischen Behandlung von Brustschmerz (Schmerzen der Brust), insbesondere hervorgerufen durch medizinische, insbesondere mammographische Untersuchungen, eignet. Dabei soll durch die vorliegende Erfindung die zuvor geschilderten Nachteile des Standes der Technik zumindest weitgehend vermeiden oder zumindest abgeschwächt werden. Neben einer guten schmerzstillenden bzw. schmerzlindernden Wirksamkeit sollten zudem eine hervorragende Verträglichkeit und eine sichere sowie einfache Anwendbarkeit gewährleistet sein.

Die Anmelderin hat in völlig überraschender Weise herausgefunden, daß die zuvor geschilderte Aufgabe dadurch gelöst werden kann, daß erfindungsgemäß eine Verwendung bereitgestellt wird, welche darauf abzieht, mindestens ein Lokalanästhetikum in Kombination mit einem topischen Analgetikum zur prophylaktischen und/oder therapeutischen Behandlung von Brustschmerz (Schmerzen der Brust), welcher insbesondere durch medizinische, insbesondere mammographische Untersuchungen hervorgerufen wird, einzusetzen. Wie zuvor angeführt, handelt es sich bei den Brustschmerzen um durch medizinische, insbesondere mammographische Untersuchungen verursachte Schmerzen, unangenehme Druck- oder Kompressionsempfindungen, Befindlichkeitsstörungen oder dergleichen der insbesondere weiblichen Brust.

Somit schlägt die vorliegende Erfindung zur Lösung der zuvor geschilderten Aufgabenstellung - gemäß einem ersten Aspekt der vorliegenden Erfindung - die Verwendung mindestens eines Lokalanästhetikums in Kombination mit einem topischen Analgetikum zur prophylaktischen und/oder therapeutischen Behandlung von Brustschmerz gemäß Anspruch 1 vor. Weitere, vorteilhafte Ausgestaltungen der erfindungsgemäßen Verwendung sind Gegenstand der diesbezüglichen Unteransprüche.

Weiterer Gegenstand der vorliegenden Erfindung - gemäß einem zweiten Aspekt der vorliegenden Erfindung - ist die topisch anwendbare Applikationsform nach der Erfindung gemäß Anspruch 13.

Schließlich ist Gegenstand der vorliegenden Erfindung - gemäß einem dritten Aspekt der vorliegenden Erfindung - ein Kit gemäß Anspruch 14.

Bei allen nachstehend genannten relativen bzw. prozentualen gewichtsbezogenen Mengenangaben ist zu beachten, daß diese im Rahmen der erfindungsgemäß eingesetzten Applikationsform bzw. Zusammensetzung derart auszuwählen sind, daß sie sich in der Summe in der erfindungsgemäß verwendeten Applikationsform bzw. Zusammensetzung unter Einbeziehung sämtlicher Komponenten bzw. Inhaltsstoffe bzw. Zusatzstoffe bzw. Bestandteile bzw. Exzipienten, insbesondere wie nachfolgend definiert, stets um 100 Gew.-% ergänzen. Dies versteht sich für den Fachmann aber von selbst. Im übrigen gilt, daß der Fachmann anwendungsbezogen oder einzelfallbedingt von den nachfolgend angeführten Mengenangaben abweichen kann, ohne daß er den Rahmen der vorliegenden Erfindung verläßt.

Es versteht sich zudem von selbst, daß Ausgestaltungen, Ausführungsformen, Vorteile und dergleichen, welche nachfolgend zu Zwecken der Vermeidung von Wiederholungen nur zu einem Erfindungsaspekt angerührt sind, selbstverständlich auch in bezug auf die übrigen Erfindungsaspekte entsprechend gelten.

Zur Lösung der vorgenannten Aufgabe wird erfindungsgemäß - gemäß einem **ersten** Aspekt der vorliegenden Erfindung - die Verwendung mindestens eines Lokalanästhetikums zur Herstellung eines Arzneimittels zur prophylaktischen und/oder therapeutischen Behandlung von durch medizinische, insbesondere mammographische Untersuchungen verursachten Schmerzen, unangenehmen Druck- oder Kompressionsempfindungen, Befindlichkeitsstörungen oder dergleichen der insbesondere weiblichen Brust, vorgeschlagen,
wobei das Lokalanästhetikum als Wirkstoff in eine topisch anwendbare Applikationsform inkorporiert wird, wobei das Lokalanästhetikum in Mengen von 1 bis 30 Gew.% in der betreffenden Applikationsform vorhanden ist, bezogen auf die Applikationsform, und wobei das Lokalanästhetikum topisch durch Aufbringen auf die Brust appliziert wird; und
wobei das Lokalanästhetikum in Kombination mit mindestens einem Analgetikum angewendet wird, wobei das Analgetikum in Mengen von 0,1 bis 30 Gew.-% in der betreffenden Applikationsform vorhanden ist, bezogen auf die Applikationsform, wobei als Analgetikum Acetylsalicylsäure, Diclofenac, Ibuprofen, Piroxicam und/oder Paracetamol eingesetzt wird und wobei das Analgetikum topisch appliziert wird.

Die Anmelderin hat in völlig überraschender Weise herausgefunden, daß die gezielte Verwendung eines Lokalanästhetikums in bezug auf mit medizinischen, insbesondere mammographischen Untersuchungen der weiblichen Brust einhergehenden Schmerzen bzw. Befindlichkeitsstörungen wirksam behandelt bzw. vermindert bzw. gelindert werden können, so daß die erfindungsgemäße Verwendung des Lokalanästhetikums eine hervorragende Wirkeffizienz aufweist. Aufgrund der erfindungsgemäßen Verwendung werden die mit mammographischen Untersuchungen einhergehenden Unannehmlichkeiten bzw. Schmerzen wirksam gelindert, so daß seitens der Patientinnen eine höhere Bereitschaft besteht, eine mammographische Untersuchung durchführen zu lassen, da die mammographische Untersuchung aufgrund der erfindungsgemäßen Verwendung des Lokalanästhetikums als weniger belastend empfunden wird. Ein weiterer Vorteil der erfindungsgemäßen Verwendung des Lokalanästhetikums ist darin zu sehen, daß eine gute Verträglichkeit vorliegt und aufgrund der topischen Applikation direkt auf die weibliche Brust eine sichere und einfache Anwendbarkeit gewährleistet ist, wobei die Patientinnen das Lokalanästhetikum auch aufgrund seiner bevorzugten Anwendungsform beispielsweise als Gel oder Salbe vor der eigentlichen Untersuchung selbst applizieren können. Die erfindungsgemäße Verwendung fokussiert zwar maßgeblich auf die prophylaktische und/oder therapeutische Behandlung von Brustschmerz der weiblichen Brust, ist jedoch nicht hierauf beschränkt, so daß auch eine Applikation bzw. Anwendung in bezug auf die männliche Brust in Betracht kommt.

Was die erfindungsgemäße Verwendung anbelangt, so ist es erfindungsgemäß vorgesehen, daß das Lokalanästhetikum topisch durch Aufbringen auf die Brust appliziert wird. Im Rahmen der erfindungsgemäßen Verwendung kann es somit vorgesehen sein, daß die Verabreichung bzw. Applikation beispielsweise durch Auf bzw. Einreiben der Brust mit der erfindungsgemäß verwendeten Applikationsform mit dem Lokalanästhetikum erfolgt.

In diesem Zusammenhang kann es erfindungsgemäß vorgesehen sein, daß das Lokalanästhetikum als Wirkstoff in eine topisch anwendbare Applikationsform inkorporiert wird. Dabei liegt die Applikationsform vorzugsweise in Form von flüssigen, viskosen oder pastösen, insbesondere semiliquiden Darreichungsformen vor, vorzugsweise in Form von Salben, Cremes, Lotionen, Gelen, Sprays oder dergleichen.

Das Lokalanästhetikum ist erfindungsgemäß in Mengen von 1 bis 30 Gew.-%, insbesondere 2 bis 20 Gew.-%, vorzugsweise 3 bis 10 Gew.-%, in der betreffenden Applikationsform vorhanden, bezogen auf die Applikationsform.

Das Lokalanästhetikum sollte bei seiner Verabreichung in wirksamen, insbesondere therapeutisch wirksamen Mengen appliziert werden. Diesbezüglich kann beispielsweise eine definierte Menge der Applikationsform, beispielsweise in Form eines Gels oder einer Salbe, auf die Brust aufgebracht und dort ver- bzw. eingerieben werden, so daß die Wirksubstanz über die Haut zum Wirkort gelangt und dort die schmerzlindernde Wirkung entfaltet. Beispielsweise können pro Brust 0,5 bis 5 g, insbesondere 1 bis 4 g, vorzugsweise 1,5 bis 3 g, der im Rahmen der erfindungsgemäßen Verwendung eingesetzten Applikationsform aufgebracht werden.

Das Lokalanästhetikum kann insbesondere in bezug auf die erfindungsgemäß verwendete Applikationsform zusammen mit mindestens einem Träger oder Exzipienten formuliert und/oder appliziert werden.

Diesbezüglich kann es im Rahmen der vorliegenden Erfindung vorgesehen sein, daß das Lokalanästhetikum zusammen mit mindestens einem üblichen pharmazeutischen Zusatz- und/oder Hilfsstoff, insbesondere aus der Gruppe von Füll-, Streck-, Binde-, Netz-, Stabilisierungs-, Färbe-, Puffer-, Riech-, Geschmacks- und/oder Konservierungsstoffen, formuliert und/oder appliziert wird.

In bezug auf das Lokalanästhetikum können eine Reihe lokalanästhetisch wirksamer Substanzen, welche dem Fachmann an sich bekannt sind, eingesetzt werden. Erfindungsgemäß ist es jedoch bevorzugt, daß das Lokalanästhetikum aus der Gruppe von Lokalanästhetika vom Estertyp, insbesondere Aminoestern, und Lokalanästhetika vom Amidtyp, insbesondere Aminoamiden, sowie deren Mischungen oder Kombination ausgewählt wird, insbesondere aus Lidocain, Mepivacain, Prilocain, Bupivacain, Articain und Ropivacain sowie deren Mischungen oder Kombination, besonders bevorzugt Lidocain.

Bei Lokalanästhetika handelt es sich im allgemeinen um Anästhetika mit örtlich begrenzter Wirkung bzw. zur örtlichen Schmerzstillung bzw. Betäubung, wobei die im Rahmen der erfindungsgemäßen Verwendung eingesetzten Lokalanästhetika keine euphorisierende oder suchterzeugende Wirkung aufweisen sollten. Die chemische Struktur aller Lokalanästhetika ist ähnlich. Sie umfaßt im allgemeinen eine lipophile aromatische Ringstruktur, eine Zwischenkette und eine hydrophile Aminogruppe. Nach der Zwischenkette unterscheidet man Aminoester ("Estertyp") und Aminoamide ("Amidtyp"). Die Aminoester werden im Gewebe durch eine Cholinesterase metabolisiert, der Abbau der Aminoamide erfolgt in der Leber durch N-Dealkylierung oder Hydrolyse.

Im Rahmen der vorliegenden Erfindung werden besonders gute Ergebnisse erreicht, wenn das Lokalanästhetikum Lidocain ist. Hierbei handelt es sich um ein Lokalanästhetikum vom Amidtyp, welches über eine gute Wirksamkeit bei gleichzeitig schnellem Wirkeintritt verfügt. Für weitere Ausführungen in bezug auf Lidocain kann beispielsweise auf Römpp Chemielexikon, 10, Auflage, Band 3, 1997, Georg Thieme Verlag Stuttgart/New York, Stichwort: "Lidocain" sowie auf die dort jeweils in bezug genommene Literatur verwiesen werden, deren gesamter Inhalt hiermit durch Bezugnahme eingeschlossen ist.

Erfindungsgemäß ist es vorgesehen, daß das Lokalanästhetikum in Kombination mit mindestens einem Analgetikum verwendet wird.

Denn die Anmelderin hat in völlig überraschender Weise herausgefunden, daß die zusätzliche Verwendung eines Analgetikums im Rahmen der vorliegenden Erfindung zu einer Wirksamkeitssteigerung hinsichtlich der therapeutischen bzw. prophylaktischen Behandlung von mit medizinischen Untersuchungen der Brust einhergehenden Schmerzen führt, wobei die Nebenwirkungen nicht signifikant erhöht sind.

Bei dem erfindungsgemäß eingesetzten Analgetikum, welches im allgemeinen von Lokalanästhetika wirkmechanistisch unterschieden werden kann, handelt es sich im allgemeinen um eine die Schmerzempfindung unterdrückende Substanz, welche das Bewußtsein, die sensorische Wahrnehmung und andere wichtige Funktionen des Zentralnervensystems jedoch nicht beeinflussen sollte.

In diesem Zusammenhang ist es vorgesehen, daß das Analgetikum topisch appliziert wird.

Was die zusätzliche Verwendung eines Analgetikums anbelangt, so kann das Analgetikum zusammen mit dem Lokalanästhetikum formuliert und/oder verabreicht werden, insbesondere in einer gemeinsamen topischen anwendbaren Applikationsform, vorzugsweise wie zuvor definiert. Hierbei kann es sich somit beispielsweise um ein Kombinationspräparat handeln, welches einerseits mindestens ein Lokalanästhetikum und andererseits mindestens ein Analgetikum in ein und derselben Formulierung, beispielsweise in einer Salbe, enthält.

Im Rahmen einer alternativen erfindungsgemäßen Ausführungsform kann es jedoch auch vorgesehen sein, daß das Analgetikum separat zu dem Lolcalanästhetikum formuliert bzw. verabreicht wird, insbesondere in einer nicht das Lokalanästhetikum enthaltenden Applikationsform,

In diesem Zusammenhang kann das Analgetikum im Rahmen der erfindungsgemäßen Verwendung in eine separate topisch anwendbare Applikationsform inkorporiert werden, insbesondere in Form von flüssigen bis viskosen oder pastösen, insbesondere semiliquiden Darreichungsformen, vorzugsweise Salben, Cremes, Lotionen, Gelen, Sprays oder dergleichen. Diesbezüglich können die wie zuvor für die Applikationsform des Lokalanästhetikums angeführten Träger bzw. Exzipienten bzw. Zusatz- und/oder Hilfsstoffe eingesetzt werden.

Auch für die erfindungsgemäße Verwendung des Analgetikums gilt gleichermaßen daß dieses in wirksamen, insbesondere therapeutisch und/oder pharmakologisch wirksamen Mengen zusammen mit mindestens einem Träger und/oder Exzipienten eingesetzt werden sollte.

In diesem Zusammenhang ist das Analgetikum in Mengen von 0,1 bis 30 Gew.-%, insbesondere 0,5 bis 20 Gew.-%, vorzugsweise 1 bis 10 Gew.-%, in der betreffenden Applikationsform vorhanden sein, bezogen auf die Applikationsform.

Erfindungsgemäß wird das Analgetikum topisch appliziert, und es können die gleichen Mengen, wie zuvor für das Lokalanästhetikum angeführt, der entsprechenden Applikationsform mit dem Analgetikum auf die Brust aufgetragen werden.

Im Rahmen der erfindungsgemäßen Verwendung können eine Vielzahl von Analgetika eingesetzt werden. So kann es im Rahmen der vorliegenden Erfindung vorgesehen sein, daß als Analgetikum ein nichtopioides Analgetikum verwendet wird.

Erfindungsgemäß ist es vorgesehen, daß als Analgetikum Acetylsalicylsäure, Diclofenac, Ibuprofen, Piroxicam und/oder Paracetamol eingesetzt wird. Insbesondere die vorgenannten Substanzen eignen sich in hervorragender Weise für eine Inkorporation in topisch verabreichbare Formulierungen, wie Gelen oder Salben.

Was die Verabreichung der im Rahmen der vorliegenden Erfindung eingesetzte(n) Applikationsform(en) anbelangt, so sollte(n) diese erfolgen derart, daß das Lokalanästhetikum und/oder das Analgetikum in wirksamen, insbesondere therapeutisch wirksamen Mengen appliziert wird.

Zur Gewährleistung einer besonders guten Wirksamkeit sollte das Lokalanästhetikum und/oder das Analgetikum insbesondere 180 Minuten bis 2 Minuten, vorzugsweise 120 Minuten bis 5 Minuten, bevorzugt 60 bis 10 Minuten, vor der medizinischen, insbesondere mammographischen Untersuchung verabreicht und oder appliziert werden.

Insbesondere sollten das Lokalanästhetikum und das Analgetikum vor der medizinischen, insbesondere mammographischen Untersuchungen ein- bis fünfmal, insbesondere ein- bis viermal, vorzugsweise ein- bis dreimal, bevorzugt ein- bis zweimal auf die Brust bzw. die Brüste aufgetragen werden, beispielsweise durch Auf- bzw. Einreiben oder dergleichen.

Wie zuvor angeführt, weist die erfindungsgemäße Verwendung eine hohe Wirkeffizienz in bezug auf Schmerzen bzw. Empfindlichkeitsstörungen der insbesondere weiblichen Brust, welche im Zusammenhang mit medizinischen, insbesondere mammographischen Untersuchungen stehen, auf, wobei im Rahmen der erfindungsgemäßen Verwendung durch die zusätzliche Verabreichung eines Analgetikums eine weitere Steigerung der Wirkeffizienz ermöglicht wird.

Insgesamt weist die erfindungsgemäße Verwendung eine hervorragende Verträglichkeit auf, insbesondere auch aufgrund der topischen Applikation zumindest des Lokalanästhetikums. Zudem weist die erfindungsgemäße Verwendung eine sehr gute Wirksamkeit auf, insbesondere da die Wirksubstanz gewissermaßen in der Nähe bzw. unmittelbar am Wirkort aufgetragen wird.

Weiterhin betrifft die vorliegende Erfindung - gemäß einem **zweiten** Aspekt der vorliegenden Erfindung - eine topisch anwendbare Applikationsform, insbesondere in Form einer flüssigen bis viskosen oder pastösen Darreichungsform, vorzugsweise Salbe, Creme, Lotion, Gel, Spray oder dergleichen, zur Verwendung in der prophylaktischen und/oder therapeutischen Behandlung von durch medizinische, insbesondere mammographische Untersuchungen verursachten Schmerzen, unangenehmen Druck- oder Kompressionsempfindungen, Befindlichkeitsstörungen oder dergleichen der insbesondere weiblichen Brust,
wobei die topisch anwendbare Applikationsform als topischen Wirkstoff mindestens ein Lokalanästhetikum in therapeutisch und/oder pharmakologisch wirksamen Mengen zusammen mit mindestens einem Träger und/öder Exzipienten enthält, wobei das Lokalanästhetikum in Mengen von 1 bis 30 Gew,-% in der betreffenden Applikationsform vorhanden ist, bezogen auf die Applikationsform, und
wobei die Applikationsform außerdem in therapeutisch und/oder pharmakologisch wirksamen Mengen mindestens ein Analgetikum enthält, wobei das Analgetikum in Mengen von 0, 1 bis 30 Gew.-% in der betreffenden Applikationsform vorhanden ist, bezogen auf die Applikationsform, enthalten ist und wobei das Analgetikum Acetylsalicylsäure, Diclofenac, Ibuprofen, Piroxicam und/oder Paracetamol ist.

Die erfindungsgemäße Applikationsform kann außerdem in wirksamen, insbesondere therapeutisch und/oder pharmakologisch wirksamen Mengen mindestens ein Analgetikum, insbesondere wie zuvor definiert, enthalten.

Schließlich betrifft die vorliegende Erfindung - gemäß einem **dritten** Aspekt der vorliegenden Erfindung - ein Kit zur Verwendung in der prophylaktischen und/oder therapeutischen Behandlung von durch medizinische, insbesondere mammographische Untersuchungen verursachte Schmerzen, unangenehmen Druck- oder Kompressionsempfindungen, Befindlichkeitsstörungen oder dergleichen der insbesondere weiblichen Brust, enthaltend
(i) mindestens eine topisch anwendbare Applikationsform, insbesondere in Form einer flüssigen bis viskosen oder pastösen Darreichungsform, vorzugsweise Salbe, Creme, Lotion, Gel, Spray oder dergleichen, wobei die topisch anwendbare Applikationsform als topischen Wirkstoff mindestens ein Lokalanästhetikum in therapeutisch und/oder pharmakologisch wirksamen Mengen zusammen mit mindestens einem Träger und/oder Exzipienten enthält, wobei das Lokalanästhetikum in Mengen von 1 bis 30 Gew.-% in der betreffenden Applikationsform vorhanden ist, bezogen auf die Applikationsform,
   und
(ii) mindestens eine separate topisch anwendbare Applikationsform, insbesondere in Form einer flüssigen bis viskosen oder pastösen Darreichungsform, vorzugsweise Salbe, Creme. Lotion, Gel, Spray oder dergleichen, wobei die Applikationsform mindestens ein Analgetikum in therapeutisch und/oder pharmakologisch wirksamen Mengen zusammen mit mindestens einem Träger und/oder Exzipienten enthält, wobei das Analgetikum in Mengen von 0,1 bis 30 Gel.-% in der betreffenden Applikationsform vorhanden ist, bezogen auf die Applikationsform, und wobei das Analgetikum Acetylsalicylsäure, Diclofenac, Ibuprofen, Piroxicam und/oder Paracetamol ist.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt. Das nachfolgende Ausführungsbeispiel dient lediglich der Veranschaulichung der vorliegenden Erfindung, ohne sich jedoch hierauf zu beschränken,

### AUSFÜHRUNGSBEISPIEL:

### Anwendungs- und Wirksamkeitsstudien:

Eine Studie mit mehr als 400 Frauen im Alter von etwa 30 bis etwa 90 Jahren, von denen mehr als 10 % angaben, in der Vergangenheit Mammographietermine wegen der Unannehmlichkeiten beim Röntgen versäumt zu haben, wurden in mehrere Gruppen unterteilt, wobei eine erste Gruppe ein Placebo-Gel, die zweite Gruppe ein Lidocain-Gel (mit einem Gehalt an Lidocain von 5 Gew.-%, bezogen auf das Gel) sowie weitere Gruppen jeweils eine Kombination des zuvor beschriebenen Lidocain-Gels mit einem Analgetikum auf Basis von Ibuprofen oder Paracetamol (jeweils topisch oder systemisch) erhielten. Die Frauen trugen das Gel bzw. die Gele 30 bis 90 min vor der Untersuchung selbst auf bzw. nahmen - sofern peroral verabreicht - das Analgetikum gleichermaßen 30 bis 90 min vor der Untersuchung zu sich. Während bei der Placebo-Gruppe keine signifikante Beeinflussung in bezug auf das Schmerzempfinden bei der mammographischen Untersuchung zu beobachten war, konnte eine deutliche Verbesserung der Schmerzsymptomatik bzw. eine deutliche Verringerung der mit der Untersuchung einhergehenden Unannehmlichkeiten bei der Gruppe mit dem erfindungsgemäßen Lidocain-Gel ermittelt werden. Die Wirkung konnte durch die zusätzliche Gabe von Ibuprofen bzw. Paracetamol weiter verbessert werden, und zwar sowohl in bezug auf die topische als auch die systemische Verabreichung des jeweiligen Analgetikums.

Die Untersuchungen zeigen insgesamt die hervorragende Wirkung der im Rahmen der erfindungsgemäßen Verwendung eingesetzten Zusammensetzung auf Basis eines Lokalanästhetikums, nämlich Lidocain, wobei die Wirkeffizienz durch die gezielte Kombination mit mindestens einem Analgetikum noch weiter verbessert werden kann. Nebenwirkungen wurden im Rahmen der durchgeführten Untersuchungen nicht beobachtet.

## Patentansprüche

1. Verwendung mindestens eines Lokalanästhetikums zur Herstellung eines Arzneimittels zur prophylaktischen und/oder therapeutischen Behandlung von durch medizinische, insbesondere mammographische Untersuchungen verursachten Schmerzen, unangenehmen Druck- oder Kompressionsempfindungen, Befindlichkeitsstörungen oder dergleichen der insbesondere weiblichen Brust,
wobei das Lokalanästhetikum als Wirkstoff in eine topisch anwendbare Applikationsform inkorporiert wird, wobei das Lokalanästhetikum in Mengen von 1 bis 30 Gew.-% in der betreffenden Applikationsform vorhanden ist, bezogen auf die Applikationsform, und wobei das Lokalanästhetikum topisch durch Aufbringen auf die Brust appliziert wird;
wobei das Lokalanästhetikum in Kombination mit mindestens einem Analgetikum angewendet wird, wobei das Analgetikum in Mengen von 0,1 bis 30 Gew.-% in der betreffenden Applikationsform vorhanden ist, bezogen auf die Applikationsform, wobei als Analgetikum Acetylsalicylsäure, Diclofenac, Ibuprofen, Piroxicam und/oder Paracetamol eingesetzt wird und wobei das Analgetikum topisch appliziert wird.

2. Verwendung nach Anspruch 1, **dadurch gekennzeichnet, daß** das Lokalanästhetikum als Wirkstoff in eine topisch anwendbare Applikationsform in Form von flüssigen bis viskosen oder pastösen, insbesondere semiliquiden Darreichungsformen, vorzugsweise Salben, Cremes, Lotionen, Gelen, Sprays oder dergleichen, inkorporiert wird.

3. Verwendung nach Anspruch 1 oder 2, **dadurch gekennzeichnet, daß** das Lokalanästhetikum in Mengen von 2 bis 20 Gew.-%; vorzugsweise 3 bis 10 Gew.-%, in der betreffenden Applikationsform vorhanden ist, bezogen auf die Applikationsform.

4. Verwendung nach einem der vorangehenden Ansprüche, **dadurch gekennzeichnet, daß** das Lokalanästhetikum aus der Gruppe von Lokalanästhetika vom Estertyp, insbesondere Aminoestern, und Lokalanästhetika vom Amidtyp, insbesondere Aminoamiden, sowie deren Mischungen oder Kombination ausgewählt ist, insbesondere aus Lidocain, Mepivacain, Prilocain, Bupivacain, Articain und Ropivacain sowie deren Mischungen oder Kombination, besonders bevorzugt Lidocain.

5. Verwendung nach einem der vorangehenden Ansprüche, **dadurch gekennzeichnet, daß** das Lokalanästhetikum Lidocain ist.

6. Verwendung nach einem der Ansprüche 1 bis 5, **dadurch gekennzeichnet, daß** das Analgetikum zusammen mit dem Lokalanästhetikum formuliert und/oder verabreicht wird, insbesondere in einer gemeinsamen topisch anwendbaren Applikationsform, vorzugsweise wie zuvor definiert

7. Verwendung nach einem der Ansprüche 1 bis 5, **dadurch gekennzeichnet, daß** das Analgetikum separat zu dem Lokalanästhetikum formuliert und/oder verabreicht wird, insbesondere in einer nicht das Lokalanästhetikum enthaltenden Applikationsform.

8. Verwendung nach Anspruch 7, wobei das Analgetikum in eine topisch anwendbare Applikationsform inkorporiert wird, insbesondere in Form von flüssigen bis viskosen oder pastösen, insbesondere semiliquiden Darreichungsformen, vorzugsweise Salben, Cremes, Lotionen, Gelen, Sprays oder dergleichen.

9. Verwendung nach einem der vorangehenden Ansprüche, **dadurch gekennzeichnet, daß** als Analgetikum in Mengen von 0,5 bis 20 Gew.-%, vorzugsweise 1 bis 10 Gew.-%, in der betreffenden Applikationsform vorhanden ist, bezogen auf die Applikationsform.

10. Verwendung nach einem der vorangehenden Ansprüche, **dadurch gekennzeichnet, daß** das Lokalanästhetikum und das Analgetikum in wirksamen, insbesondere therapeutisch wirksamen Mengen appliziert werden.

11. Verwendung nach einem der vorangehenden Ansprüche, **dadurch gekennzeichnet, daß** das Lokalanästhetikum und/oder das Analgetikum insbesondere 180 Minuten bis 2 Minuten, vorzugsweise 120 Minuten bis 5 Minuten, bevorzugt 60 bis 10 Minuten, vor der medizinischen, insbesondere mammographischen Untersuchung verabreicht und oder appliziert wird.

12. Verwendung nach einem der vorangehenden Ansprüche, **dadurch gekennzeichnet, daß** das Lokalanästhetikum und/oder das Analgetikum vor der medizinischen, insbesondere mammographischen Untersuchungen ein- bis fünfmal, insbesondere ein- bis viermal, vorzugsweise ein- bis dreimal, bevorzugt ein- bis zweimal auf die Brust bzw. Brüste aufgetragen wird.

13. Topisch anwendbare Applikationsform, insbesondere in Form einer flüssigen bis viskosen oder pastösen Darreichungsform, vorzugsweise Salbe, Creme, Lotion, Gel, Spray oder dergleichen, zur Verwendung in der prophylaktischen und/oder therapeutischen Behandlung von durch medizinische, insbesondere mammographische Untersuchungen verursachten Schmerzen, unangenehmen Druck-oder Kompressionsempfindungen, Befindlichkeitsstörungen oder dergleichen der insbesondere weiblichen Brust,
wobei die topisch anwendbare Applikationsform als topischen Wirkstoff mindestens ein Lokalanästhetikum in therapeutisch und/oder pharmakologisch wirksamen Mengen zusammen mit mindestens einem Träger und/oder Exzipienten enthält, wobei das Lokalanästhetikum in Mengen von 1 bis 30 Gew.-% in der betreffenden Applikationsform vorhanden ist, bezogen auf die Applikationsform, und
wobei die Applikationsform außerdem in therapeutisch und/oder pharmakologisch wirksamen Mengen mindestens ein Analgetikum enthält, wobei das Analgetikum in Mengen von 0,1 bis 30 Gew.-% in der betreffenden Applikationsform vorhanden ist, bezogen auf die Applikationsform, enthalten ist und wobei das Analgetikum Acetylsalicylsäure, Diclofenac, Ibuprofen, Piroxicam und/oder Paracetamol ist.

14. Kit zur Verwendung in der prophylaktischen und/oder therapeutischen Behandlung von durch medizinische, insbesondere mammographische Untersuchungen verursachte Schmerzen, unangenehmen Druck- oder Kompressionsempfindungen, Befindlichkeitsstörungen oder dergleichen der insbesondere weiblichen Brust, enthaltend
(i) mindestens eine topisch anwendbare Applikationsform, insbesondere in Form einer flüssigen bis viskosen oder pastösen Darreichungsform, vorzugsweise Salbe, Creme, Lotion, Gel, Spray oder dergleichen, wobei die topisch anwendbare Applikationsform als topischen Wirkstoff mindestens ein Lokalanästhetikum in therapeutisch und/oder pharmakologisch wirksamen Mengen zusammen mit mindestens einem Träger und/oder Exzipienten enthält, wobei das Lokalanästhetikum in Mengen von 1 bis 30 Gew.-% in der betreffenden Applikationsform vorhanden ist, bezogen auf die Applikationsform,
und
(ii) mindestens eine separate topisch anwendbare Applikationsform, insbesondere in Form einer flüssigen bis viskosen oder pastösen Darreichungsform, vorzugsweise Salbe, Creme, Lotion, Gel, Spray oder dergleichen, wobei die Applikationsform mindestens ein Analgetikum in therapeutisch und/oder pharmakologisch wirksamen Mengen zusammen mit mindestens einem Träger und/oder Exzipienten enthält, wobei das Analgetikum in Mengen von 0,1 bis 30 Gew.-% in der betreffenden Applikationsform vorhanden ist, bezogen auf die Applikationsform, und wobei das Analgetikum Acetylsalicylsäure, Diclofenac, Ibuprofen, Piroxicam und/oder Paracetamol ist.

## Claims

1. A use of at least one local anesthetic for producing a pharmaceutical for the prophylactic and/or therapeutic treatment of pain caused by medical, in particular mammographic examinations, uncomfortable pressure or compression sensations, discomfort, or the like, in particular of the female breast,
wherein the local anesthetic as an active ingredient is incorporated into a topically usable application form, wherein the local anesthetic is present in the respective application form in amounts of 1 to 30% by weight, based on the application form, and wherein the local anesthetic is applied topically by placing it on the breast;
wherein the local anesthetic is utilized in combination with at least one analgetic, wherein the analgetic is present in the respective application form in amounts of 0.1 to 30% by weight, based on the application form, wherein the analgetic is utilized as acetylsalicylic acid, Diclofenac, lbuprofen, Piroxicam, and/or Paracetamol, and wherein the analgetic is applied topically.

2. The use according to claim 1, **characterized in that** the local anesthetic as an active ingredient is incorporated into a topically usable application form in the form of liquid to viscous, or paste-like, in particular semi-liquid dosage forms, preferably ointments, creams, lotions, gels, sprays, or the like.

3. The use according to claims 1 or 2, **characterized in that** the local anesthetic is present in the respective application form at amounts of 2 to 20% by weight, preferably 3 to 10% by weight, based on the application form.

4. The use according to one of the previous claims, **characterized in that** the local anesthetic is selected from the group consisting of local anesthetics of the ester type, particularly amino esters, and local anesthetics of the amide type, particularly amino amides, as well as the mixtures or combinations thereof, in particular from Lidocain, Mepivacain, Prilocain, Bupivacain, Articain, and Ropivacain, as well as the mixtures or combinations thereof, particularly preferred Lidocain.

5. The use according to one of the previous claims, **characterized in that** the local anesthetic is Lidocain.

6. The use according to one of the claims 1 to 5, **characterized in that** the analgetic is formulated and/or administered together with the local anesthetic, in particular in a mutually topically usable application form, preferably as defined above.

7. The use according to one of the claims 1 to 5, **characterized in that** the analgetic is formulated and/or administered separately from the local anesthetic, in particular in an application form that does not contain the local anesthetic.

8. The use according to claim 7, wherein the analgetic is incorporated into a topically usable application form, in particular in the form of liquid to viscous, or paste-like, in particular semi-liquid dosage forms, preferably ointments, creams, lotions, gels, sprays, or the like.

9. The use according to one of the previous claims, **characterized in that** the analgetic is present in the respective application form in amounts of 0.5 to 20% by weight, preferably 1 to 10% by weight, based on the application form.

10. The use according to one of the previous claims, **characterized in that** the local anesthetic and the analgetic are applied in effective, particularly therapeutically effective amounts.

11. The use according to one of the previous claims, **characterized in that** the local anesthetic and/or the analgetic are administered and/or applied in particular 180 minutes to 2 minutes, preferably 120 minutes to 5 minutes, more preferred 60 to 10 minutes, before the medical, in particular mammographic examination.

12. The use according to one of the previous claims, **characterized in that** the local anesthetic and/or the analgetic is applied to the breast, or breasts, respectively, before the medical, in particular mammographic examination one to five times, in particular one to four times, preferably one to three times, more preferred one to two times.

13. A topically usable application form, in particular in the form of a liquid to viscous, or paste-like dosage form, preferably an ointment, cream, lotion, gel, spray, or the like, for use in the prophylactic and/or therapeutic treatment of pain caused by medical, in particular mammographic examinations, uncomfortable pressure or compression sensations, discomfort, or the like, in particular of the female breast,
wherein the topically usable application form as the topical active ingredient has at least one local anesthetic in therapeutically and/or pharmacologically effective amounts, together with at least one carrier and/or excipient, wherein the local anesthetic is present in the respective application form in amounts of 1 to 30% by weight, based on the application form, and
wherein the application form further has at least one analgetic in therapeutically and/or pharmacologically effective amounts, wherein the analgetic is comprised in the respective application form in amounts of 0.1 to 30% by weight, based on the application form, and wherein the analgetic is acetylsalicylic acid, Diclofenac, lbuprofen, Piroxicam, and/or Paracetamol.

14. A kit for use in the prophylactic and/or therapeutic treatment of pain caused by medical, in particular mammographic examinations, uncomfortable pressure or compression sensations, discomfort, or the like, in particular of the female breast, comprising
(i) at least one topically usable application form, in particular in the form of a liquid to viscous, or paste-like dosage form, preferably an ointment, cream, lotion, gel, spray, or the like, wherein the topically usable application form contains as the active ingredient at least one local anesthetic in therapeutically and/or pharmacologically effective amounts, together with at least one carrier and/or excipient, wherein the local anesthetic is present in the respective application form in amounts of 1 to 30% by weight, based on the application form,
and
(ii) at least one separate, topically usable application form, in particular in the form of a liquid to viscous, or paste-like dosage form, preferably an ointment, cream, lotion, gel, spray, or the like, wherein the application form comprises at least one analgetic in therapeutically and/or pharmacologically effective amounts, together with at least one carrier and/or excipient, wherein the analgetic is present in the respective application form in amounts of 0.1 to 30% by weight, based on the application form, and wherein the analgetic is acetylsalicylic acid, Diclofenac, lbuprofen, Piroxicam, and/or Paracetamol.

## Revendications

1. Utilisation d'au moins un anesthésique local à la préparation d'un médicament pour le traitement prophylactique et/ou thérapeutique de douleurs, de sensations de pression ou de compression, de malaises ou similaires, en particulier du sein féminin, provoqués par des examens médicaux, en particulier mammographiques,
l'anesthésique local étant incorporé comme principe actif dans une forme d'application utilisable topiquement, l'anesthésique local étant présent dans la forme d'application en question en des quantités de 1 à 30 % en poids, rapportées à la forme d'application et l'anesthésique local étant appliqué topiquement par application sur le sein ;
l'anesthésique local étant utilisé en combinaison avec au moins un analgésique, l'analgésique étant présent dans la forme d'application considérée en des quantités de 0,1 à 30 % en poids, rapportées à ladite forme d'application, sachant que comme analgésique sont utilisés l'acide acétylsalicylique, le diclofénac, l'ibuprofène, le piroxicam et/ou le paracétamol, et que l'analgésique est appliqué topiquement.

2. Utilisation selon la revendication 1, **caractérisée en ce que** l'anesthésique local est incorporé comme principe actif dans une forme d'application applicable topiquement sous forme liquide à visqueuse ou pâteuse, en particulier dans des formes d'administration semi-liquides, de préférence des pommades, des crèmes, des lotions, des gelées, des sprays ou similaires.

3. Utilisation selon la revendication 1 ou 2, **caractérisée en ce que** l'anesthésique local est présent dans la forme d'application concernée en des quantités de 2 à 20 % en poids, de préférence de 3 à 10 % en poids, rapportées à la forme d'application.

4. Utilisation selon l'une quelconque des revendications précédentes, **caractérisée en ce que** l'anesthésique local est choisi parmi le groupe des anesthésiques locaux de type ester, en particulier des aminoesters, et des anesthésiques locaux du type amide, en particulier des aminoamides, ainsi que parmi leurs mélanges et leurs combinaisons, en particulier de la lidocaïne, de la mépivacaïne, de la prilocaïne, de la bupivacaïne, de l'articaïne et de la ropivacaïne, ainsi que de leurs mélanges et de leurs combinaisons, la lidocaïne étant particulièrement préférée.

5. Utilisation selon l'une quelconque des revendications précédentes, **caractérisée en ce que** l'anesthésique local est la lidocaïne.

6. Utilisation selon l'une quelconque des revendications 1 à 5, **caractérisée en ce que** l'analgésique est formulé et/ou administré ensemble avec l'anesthésique local, en particulier dans une forme commune applicable topiquement, de préférence comme définie précédemment.

7. Utilisation selon l'une quelconque des revendications 1 à 5, **caractérisée en ce que** l'analgésique est formulé et/ou administré séparément de l'anesthésique local, en particulier sous une forme d'application ne contenant pas l'anesthésique local.

8. Utilisation selon la revendication 7, **caractérisée en ce que** l'analgésique est incorporé dans une forme d'application applicable topiquement, en particulier sous des formes d'administration liquides à visqueuses ou pâteuses, en particulier semi-liquides, de préférence des pommades, des crèmes, des lotions, des gelées, des sprays ou similaires.

9. Utilisation selon l'une quelconque des revendications précédentes, **caractérisée en ce que** l'analgésique est présent dans la forme d'application concernée en des quantité de 0,5 à 20 % en poids, de préférence de 1 à 10 % en poids, rapportées à la forme d'application.

10. Utilisation selon l'une quelconque des revendications précédentes, **caractérisée en ce que** l'anesthésique local et l'analgésique sont appliqués dans des quantités efficaces, en particulier thérapeutiquement efficaces.

11. Utilisation selon l'une quelconque des revendications précédentes, **caractérisée en ce que** l'anesthésique local et/ou l'analgésique sont administrés et/ou appliqués en particulier 180 minutes à 2 minutes, de préférence 180 minutes à 5 minutes, plus préférablement de 60 à 10 minutes avant l'examen médical, en particulier mammographique.

12. Utilisation selon l'une quelconque des revendications précédentes, **caractérisée en ce que** l'anesthésique local et/ou l'analgésique sont appliqués une à cinq fois, en particulier une à quatre fois, de préférence une à trois fois, encore plus préférablement une à deux fois sur le ou les seins avant l'examen médical, en particulier mammographique.

13. Forme d'application topique, en particulier sous la forme d'une forme d'administration liquide à visqueuse ou pâteuse, en particulier semi-liquide, de préférence de pommade, de crème, de lotion, de gelée, de spray ou similaires, pour une utilisation dans le traitement prophylactique et/ou thérapeutique de douleurs, de sensations de pression ou de compression, de malaises ou similaires, en particulier du sein féminin, provoqués par des examens médicaux, en particulier mammographiques,
la forme d'application utilisable topiquement contenant comme principe actif topique au moins un anesthésique local dans des quantités thérapeutiquement et/ou pharmacologiquement efficaces, ensemble avec au moins un vecteur et/ou excipient, l'anesthésique local étant présent dans la forme d'application concernée en des quantités de 1 à 30 % en poids, rapportées à la forme d'application, et
la forme d'application contenant en outre dans des quantités thérapeutiquement et/ou pharmacologiquement efficaces au moins un analgésique, l'analgésique étant présent dans la forme d'application concernée dans des quantités de 0,1 à 30 % en poids, rapportées à la forme d'application, et l'analgésique étant de l'acide acétylsalicylique, du diclofénac, de l'ibuprofène, du piroxicam et/ou du paracétamol.

14. Kit pour l'utilisation dans le traitement prophylactique et/ou thérapeutique de douleurs, de sensations de pression ou de compression, de malaises ou similaires, en particulier du sein féminin, provoqués par des examens médicaux, en particulier mammographiques, contenant
(i) au moins une forme d'application utilisable topiquement, en particulier sous forme de formes d'administration liquides à visqueuses ou pâteuses, de préférence de pommades, de crèmes, de lotions, de gelées, de sprays ou similaires, la forme d'application utilisable topiquement contenant comme principe actif topique au moins un anesthésique local dans des quantités thérapeutiquement et/ou pharmacologiquement efficaces, ensemble avec au moins un vecteur et/ou un excipient, l'anesthésique local étant présent dans la forme d'application concernée dans des quantités de 1 à 30 % en poids, rapportées à la forme d'application,
et
(ii) au moins une forme d'application séparée utilisable topiquement, en particulier sous forme de formes d'administration liquides à visqueuses ou pâteuses, de préférence de pommades, de crèmes, de lotions, de gelées, de sprays ou similaires, la forme d'application contenant comme principe actif topique au moins un analgésique dans des quantités thérapeutiquement et/ou pharmacologiquement efficaces, ensemble avec au moins un vecteur et/ou un excipient, l'analgésique étant présent dans la forme d'application concernée dans des quantités de 0,1 à 30 % en poids, rapportées à la forme d'application, l'analgésique étant de l'acide acétylsalicylique, du diclofénac, de l'ibuprofène, du piroxicam et/ou du paracétamol.
